# EUROPÄISCHE PATENTANMELDUNG

(11) **EP 1 139 692 A2**
(43) Veröffentlichungstag der Anmeldung: **04.10.2001**
(21) Anmeldenummer: 00119192.3
(22) Anmeldetag: 05.09.2000
(51) Int. Cl.: H04R 25/00

(54) **Teil- oder vollimplantierbares Hörsystem**

(30) Priorität: 28.03.2000 DE 10015421
(71) Anmelder: IMPLEX Aktiengesellschaft Hearing Technology, 85737 Ismaning (DE)
(72) Erfinder: Leysieffer, Hans, Dr.-Ing., 82024 Taufkirchen (DE); Rinser, Günter, 83620 Feldkirchen (DE)
(74) Vertreter: Schwan, Gerhard, Dipl.-Ing.

(57) **Zusammenfassung**

Die Erfindung betrifft ein teil- oder vollimplantierbares Hörsystem, das mit einer Einheit (14, 30; 460) für die elektromechanische Stimulation des Mittel- oder Innenohres oder die direkte elektrische Stimulation des Innenohres versehen ist. Das Hörsystem umfasst ferner eine Einrichtung (26, 126, 154, 526) zur direkten Anbindung des Hörsystems an Telekommunikationsnetze ohne die Verwendung von Telekommunikationsendgeräten.

## Beschreibung

Die vorliegende Erfindung betrifft ein teil- oder vollimplantierbares Hörsystem gemäß dem Oberbegriff von Anspruch 1.

Unter dem Begriff "Hörstörung" sollen vorliegend Innenohrschäden, Mittelohrschäden, kombinierte Innenohr- und Mittelohrschäden, cochleäre Taubheit, die den Einsatz eines Cochlea-Implantats notwendig macht, ebenso wie retrocochleare Hörstörungen, die den Einsatz eines Hirnstamm-Implantats notwendig machen, verstanden werden, d.h. kurz alles was die Schallaufnahme und/oder die Weiterleitung bis zum Hirnstamm verhindert oder beeinträchtigt.

Die Rehabilitation sensorischer Hörstörungen mit teilimplantierbaren, elektronischen Systemen hat in den letzten Jahren einen bedeutenden Stellenwert erhalten. Insbesondere gilt dies für den Patientenkreis, bei dem das Gehör durch Unfall, Krankheit oder sonstige Einflüsse vollständig ausgefallen oder bereits von Geburt an nicht funktionsfähig ist. Ist in diesen Fällen nur das Innenohr (Cochlea) und nicht die nach zentral führende neuronale Hörbahn betroffen, kann mit elektrischen Reizsignalen der verbliebene Hörnerv stimuliert und somit ein Höreindruck erzeugt werden, der bis zu einem offenen Sprachverständnis führen kann. Bei diesen sogenannten Cochlea Implantaten wird ein Reizelektroden-Array in die Cochlea eingeführt, das von einem elektronischen System angesteuert wird, wobei dieses hermetisch dichte und biokompatibel eingekapselte Elektronikmodul operativ im knöchernen Bereich hinter dem Ohr (Mastoid) eingebettet ist. Das elektronische System enthält jedoch im wesentlichen nur Dekodier- und Treiberschaltungen für die Reizelektroden; die akustische Schallaufnahme, die Wandlung dieses Schallsignals in elektrische Signale und deren weitere Verarbeitung erfolgt grundsätzlich extern in einem sogenannten Sprachprozessor, der außen am Körper getragen wird. Der Sprachprozessor setzt die vorverarbeiteten Signale entsprechend kodiert auf ein hochfrequentes Trägersignal um, das über eine induktive Kopplung durch die geschlossene Haut (transkutan) zu dem Implantat übertragen wird. Das schallaufnehmende Mikrofon befindet sich ausnahmslos außerhalb des Körpers und in den meisten Anwendungen in einem an der Ohrmuschel getragenen Gehäuse eines Hinter-dem-Ohr-Hörgerätes (HdO) und ist mit einem Kabel mit dem Sprachprozessor verbunden. Solche Cochlea-Implantat-Systeme, deren Komponenten und Prinzipien der transkutanen Signalübertragung sind beispielhaft in US-A-5 070 535, US-A-4 441 210, EP-A-0 200 321 und US-A-5 626 629 beschrieben. Verfahren zur Sprachaufbereitung und -kodierung bei Cochlea-Implantaten sind beispielsweise in EP-A-0 823 188, EP-B-0 190 836, US-A-5 597 380, US-A-5 271 397, US-A-5 095 904, US-A-5 601 617 und US-A-5 603 726 angegeben.

Neben der Rehabilitation gehörloser bzw. ertaubter Patienten mit Cochlea-Implantaten existieren seit geraumer Zeit Ansätze, Patienten mit einer sensorineuralen Hörstörung, die operativ nicht behebbar ist, mit teil- bzw. vollimplantierbaren Hörgeräten eine bessere Rehabilitation als mit konventionellen Hörgeräten zu bieten. Das Prinzip besteht in den überwiegenden Ausführungsformen darin, ein Ossikel des Mittelohres oder das Innenohr direkt über einen mechanischen bzw. hydromechanischen Reiz zu stimulieren und nicht über das verstärkte akustische Signal eines konventionellen Hörgerätes, bei dem das verstärkte Schallsignal dem äußeren Gehörgang zugeführt wird. Der aktorische Stimulus dieser elektromechanischen Systeme wird mit verschiedenen physikalischen Wandlerprinzipien realisiert wie z.B. durch elektromagnetische und piezoelektrische Systeme. Der Vorteil dieser Verfahren wird hauptsächlich in der gegenüber konventionellen Hörgeräten verbesserten Klangqualität und bei vollimplantierten Systemen in der Unsichtbarkeit der Hörprothese gesehen. Solche teil- und vollimplantierbaren elektromechanischen Hörgeräte sind beispielhaft von Yanigahara et al. in Arch Otolaryngol Head Neck, Surg-Vol 113, August 1987, S. 869-872, Suzuki et al. in Advances in Audiology, Vol. 4, Karger Basel, 1988, Leysieffer et al. in HNO Vol. 46, 1998, S. 853-863, Zenner et al. in HNO Vol. 46, 1998, S. 844-852 und in zahlreichen Patentdokumenten beschrieben, insbesondere in US-A-5 999 632, US-A-5 277 694, US-A-5 411 467, US-A-3 764 748, US-A-4 352 960, US-A-5 015 224, US-A-5 015 225, US-A-3 557 775, US-A-3 712,962, US-A-4 729 366, US-A-4 988 333, EP-B-0 263 254, US-A-5 814 095, US-A-4 850 962, WO-A-98/36711, WO-A-98/06237, US-A-5 859 916, WO-A-98/03035, WO-A-99/08481, WO-A-99/08475, WO-A-99/07436 und in WO-A-97/18689.

Bei allen vorstehend genannten Rehabilitationsgeräten erscheint es heute als sehr sinnvoll, die Systeme so auszulegen, daß sie vollständig implantiert werden können. Solche Hörsysteme bestehen je nach angestrebter Funktion aus drei oder aus vier Funktionseinheiten: einem Sensor (Mikrofon), der den einfallenden Luftschall in ein elektrisches Signal umwandelt, einer elektronischen Signalverarbeitungs-, Verstärkungs- und Implantatsteuerungseinheit, einem elektromechanischen bzw. implantierbaren elektroakustischen Wandler, der die verstärkten und vorverarbeiteten Sensorsignale in mechanische bzw. akustische Schwingungen umwandelt und diese über geeignete Koppelmechanismen dem geschädigten Mittel- und/oder Innenohr zuführt oder einer cochleären Reizelektrode bei Cochlea-Implantaten sowie einem elektrischen Energieversorgungssystem, das die genannten Module versorgt. Weiterhin kann eine externe Einheit vorgesehen sein, die dem Implantat elektrische Nachladeenergie zur Verfügung stellt, wenn die implantatseitige Energieversorgungseinheit eine nachladbare (Sekundär-)Batterie enthält. Besonders vorteilhafte Vorrichtungen und Verfahren zum Laden von nachladbaren Implantatbatterien sind in DE-A-198 38 137 sowie in US-A-5 279 292 beschrieben. Zweckmäßig ist auch eine Telemetrieeinheit vorzusehen, mit der patientenspezifische, audiologische Daten drahtlos bidirektional übertragen bzw. im Implantat programmiert und damit dauerhaft gespeichert werden können, wie es von Leysieffer et al. in HNO Vol. 46, 1998, S. 853-863 beschrieben wurde.

Um eine einfache Aktualisierung der Betriebssoftware des Implantats ohne chirurgischen Eingriff zu ermöglichen, wird in der älteren EP Patentanmeldung 99 115 256.2 vorgeschlagen, die Elektronik des Implantats so zu gestalten, daß mindestens Teile des Betriebsprogramms durch von einer externen Einheit über eine Telemetrieeinrichtung, die vorzugsweise induktiv arbeitet, übermittelte Daten geändert oder ausgetauscht werden können.

Ein Aspekt bei Hörsystemen ist deren Verwendung im Zusammenhang mit Telekommunikationseinrichtungen.

WO 98/51124 betrifft die Anpassung von Telekommunikationsendgeräten, wie beispielsweise dem Mobilteil eines schnurlosen Telefons, an Hörsysteme zur Rehabilitierung eines Hörschadens. Dabei wird vorgeschlagen, das Mobilteil mit einer Einheit auszustatten, welche das an den Lautsprecher des Mobilteils gelieferte Signal in Abhängigkeit von Informationen bezüglich des von dem Anwender benutzten Hörsystems gegenüber dem Normalbetrieb für einen Nichthörgeschädigten dergestalt abwandelt, daß der Benutzer des Hörsystems einen möglichst optimalen Höreindruck erhält. Bei den Hörgeräten handelt es sich um herkömmliche elektroakustische Geräte oder um ein Cochlea-Implantat, bei welchem das Mikrofon und der Sprachprozessor äußerlich hinter der Ohrmuschel getragen werden. In letzterem Fall wird vorgeschlagen, das von dem Telekommunikationsendgerät speziell für das verwendete Hörgerät aufbereitete Signal direkt dem Empfänger des Cochlea-Implantats oder dem implantierten Elektrodendraht zuzuführen, wobei für den letzteren Fall die Verwendung einer Buchse oder eines entsprechenden Buses vorgeschlagen wird. Nachteilig bei diesem Hörsystem ist, daß speziell modifzierte Telekommunikationsendgeräte verwendet werden müssen, um dem Hörsystembenutzer die Nutzung von Telekommunikationsnetzen zu ermöglichen.

Aus US 5 824 022 ist ein Cochlea-Implantatsystem bekannt, welches einen implantierbaren Cochlea-Stimulator und eine hinter dem Ohr getragene Sprachprozessoreinheit aufweist, welche einerseits über ein Mikrofon aufgefangene Schallsignale verarbeitet und induktiv zu dem implantierbaren Cochlea-Stimulator überträgt, und andererseits über eine HF-Strecke bidirektional mit einer Fernbedienung kommunizieren kann, welche eine Audiobuchse zur Aufnahme von Radio-, TV- und Walkman-Audiosignalen aufweist, welche mittels eines Audioprozessors verarbeitet und an die Sprachprozessoreinheit gesendet werden können.

Aus US 6 021 207 ist ein Ohrhörer bekannt, welcher in den Ohrkanal eingesetzt werden kann und ein Mikrofon sowie einen Lautsprecher aufweist. Der Ohrhörer steht in drahtloser bidirektionaler Kommunikation mit einer Fernbedienungseinheit, um einerseits von dem Mikrofon erfaßte Audiosignale an diese zu senden und andererseits Audiosignale von dieser zu empfangen und über den Lautsprecher auszugeben. Bei der Fernbedienung kann es sich um ein Mobiltelefon bzw. um eine daran angeschlossene Einheit handeln.

Aus EP 0 671 818 A1 ist ein HF-Empfänger bekannt, der inklusive Batterie und Hörer in einem Gehäuse angeordnet werden kann, das in den Gehörgang einer Person einfügbar ist.

Es ist Aufgabe der vorliegenden Erfindung, ein teil- oder vollimplantierbares Hörsystem zu schaffen, welches seinem Benutzer auf einfache Weise die Nutzung von Telekommunikationsnetzen ermöglicht.

Diese Aufgabe wird erfindungsgemäß gelöst durch ein teil- oder vollimplantierbares Hörsystem, wie es in Anspruch 1 definiert ist. Bei dieser erfindungsgemäßen Lösung ist vorteilhaft, daß eine Anbindung des Hörsystems nicht über Telekommunikationsendgeräte erfolgt, die entweder in aufwendiger Weise speziell auf das verwendete Hörsystem abgestimmt werden müssen oder, falls eine solche Anpassung unterlassen wird, wegen der dann mangelnden Abstimmung zwischen Hörsystem und Telekommunikationsendgerät nur die Erzielung einer mangelhaften Hörqualität erlauben, sondern daß die Anbindung durch eine Modifikation des Hörsystems selbst erzielt wird. Ferner entfällt auf diese Weise überhaupt die Verwendung eines Telekommunikationsendgeräts, was eine wesentliche Erleichterung für den Hörsystembenutzer mit sich bringt, da er kein solches Endgerät mehr mit sich führen muß und dadurch z.B. die Hände jederzeit frei hat. Auch kann dadurch der Empfang von Sprachnachrichten in für Dritte nicht sichtbarer und vor allem nicht hörbarer Weise erfolgen, so daß Dritte beispielsweise nicht gestört werden. Der Schallaufnehmer und die Stimulationseinheit des Hörsystems ersetzen dabei funktionsmäßig die entsprechenden Komponenten (Mikrofon und Hörer) eines Telekommunikationsendgeräts.

Grundsätzlich kann das Hörsystem für eine Rehabilitation einer Hörstörung des Benutzers oder aber auch für einen Benutzer ohne Hörstörung ausgelegt sein. In letzterem Fall wird bei einer Vollimplantation ein "unsichtbares Mobiltelefon" realisiert.

Bevorzugte Ausgestaltungen der Erfindung ergeben sich aus den Unteransprüchen.

Im folgenden werden Ausführungsformen der Erfindung anhand der beigefügten Zeichnungen beispielhaft näher erläutert, wobei:
Fig. 1 schematisch ein erfindungsgemäßes Hörsystem im implantierten Zustand, teilweise in Schnittdarstellung, im Zusammenwirken mit einem Telekommunikationsnetz zeigt; und
Fign. 2 bis 10 Ansichten wie Fig. 1 zeigen, wobei jedoch andere Ausführungsformen der Erfindung dargestellt sind.

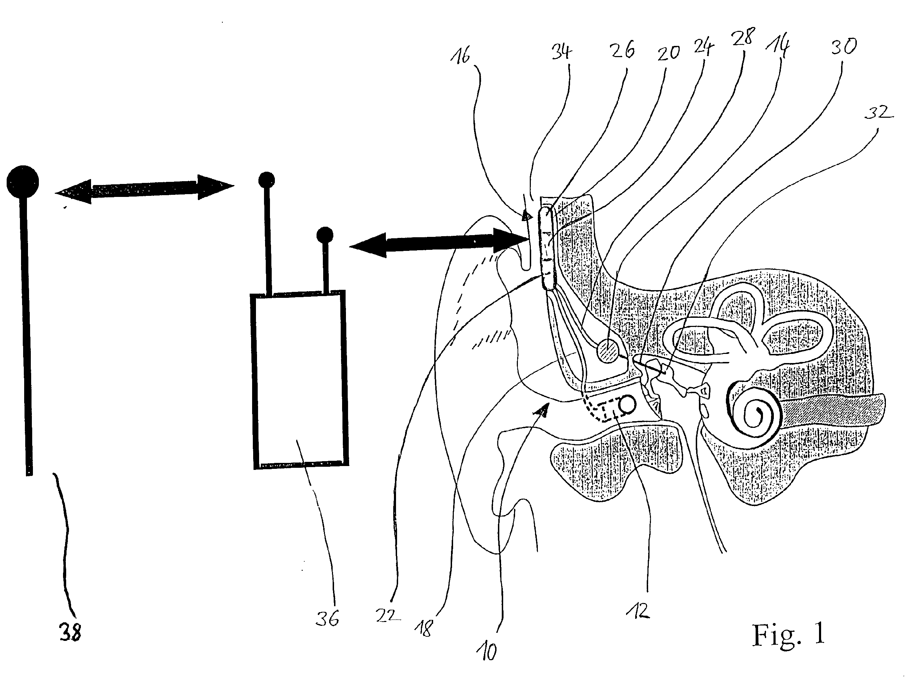

Fig. 1 zeigt in teilweiser Schnittdarstellung ein voll implantierbares Hörsystem 10, welches als wesentliche Komponenten einen Schallaufnehmer 12 (Mikrofon), einen elektromechanischen Wandler 14 sowie ein Elektronikmodul 16 umfaßt. Das Mikrofon 12, das. in der hinteren Gehörgangswand subkutan implantiert ist, nimmt den Schall auf und wandelt ihn in ein elektrisches Signal um, das über die Implantatleitung 18 dem Elektronikmodul 16 zugeführt wird. Das Elektronikmodul 16 ist in einem hermetisch dichten und biokompatiblen Implantatgehäuse 20 untergebracht, welches in einem künstlichen Knochenbett im Mastoidbereich des Schädels implantiert ist, und umfaßt eine Signalbearbeitungs/Steuereinheit 22, einen wideraufladbaren elektrischen Energiespeicher 24 und einen HF-Sender/Empfänger 26. Die Signalbearbeitungs/Steuereinheit 22 ist in beispielhafter Ausgestaltung in der eingangs erwähnten älteren EP-Patentanmeldung 99 115 256.2 beschrieben und dient dazu, den Betrieb des Hörsystems zu steuern und von dem Mikrofon 12 erzeugte elektrische Signale so zu bearbeiten, daß sie über eine implantierbare Leitung 28 dem elektromechanischen Wandler 14 als Eingangssignale zugeführt werden können. Die ausgangsseitigen mechanischen Schwingungen des Wandlers 14 werden über ein geeignetes Koppelelement 30 direkt auf ein Ossikel der Mittelohrkette übertragen, im dargestellten Fall auf den Amboß 32. Die dort eingekoppelten Wandlerschwingungen gelangen über die Ossikelkette zum Innenohr und rufen dort einen entsprechenden Höreindruck hervor. Geeignete Koppelelemente sind beispielhaft in US-A-5 941 814 beschrieben, während vorteilhafte Wandler beispielsweise unter anderem in US-A-5 277 694 und EP-A-0 984 665, EP-A-0 984 663 und EP-A-1 011 295 beschrieben sind. Ein geeignetes Mikrofon 12 ist in US-A-5 814 095 beschrieben.

Der Energiespeicher 24 ist vorzugsweise wiederaufladbar ausgebildet, wobei das Aufladen über eine nicht dargestellte Energieempfangsschaltung zur implantatseitigen Bereitstellung von Nachladeenergie, vorzugsweise auf induktivem Weg, im Zusammenspiel mit einer externen drahtlosen Ladeeinheit erfolgen kann, wie dies beispielsweise in DE-A-198 37 913 beschrieben ist.

Abgesehen von dem HF-Sender/Empfänger 26 sind die bisher beschriebenen Komponenten des Hörsystems an sich bekannt. Der HF-Sender/Empfänger 26 ist vorgesehen, um durch die geschlossene Haut 34 hindurch mit einem in örtlicher Nähe stationierten HF-Interface 36 bidirektional zu kommunizieren. Der maximale Abstand zu dem Hörsystem 10 kann dabei wie bei drahtlosen Haustelefonen etwa 300 m betragen. Der stationäre Repeater 36, der aufgrund der begrenzten Sendeleistung des HF-Sender/Empfängers 26 erforderlich ist, wird netzgebunden mit Energie versorgt und steht in bidirektionaler Kommunikation mit einem HF-Datennetzknotenpunkt 38 (stationärer Großdistanz-HF-Sender/Empfänger), wie dies bei Mobiltelefonnetzen bekannt ist. Mit dem Begriff "Großdistanz" sind vorliegend Distanzen bis zum üblichen Abstand von HF-Datennetzknotenpunkten gemeint. Mit "Kleindistanz" sind Distanzen gemeint, wie sie beispielsweise bei drahtlosen Haustelefonen zulässig sind (bis zu ca. 300 m).

Bei einem eingehenden Anruf beispielsweise wird das Sprachsignal von dem Großdistanzsender 38 auf den stationären Kleindistanz-Repeater 36 und von diesem durch die Haut 34 auf den implantierten HF-Sender/Empfänger 26 übertragen, wo es empfangen, demoduliert und als (niederfrequentes) Audiosignal in den Audiosignalpfad des Hörsystems 10 eingespeist wird, so daß dieses Signal, bei dem es sich beispielsweise um die Stimme des Telefonpartners handelt, implantatseitig hörbar wird. Das Implantatmikrofon 12, welches im Beispiel subkutan im tiefen knöchernen Gehörgang implantiert ist, nimmt die Stimme/Sprache des Hörsystemnutzers auf, wandelt sie in ein elektrisches Signal und führt es dem Elektronikmodul 16 zu. Hier wird das Mikrofonsignal auf einen HF-Träger moduliert und von dem Sender/Empfänger 26 durch die Haut 34 zu dem Repeater 36 gefunkt, welcher es an den stationären Großdistanz-Sender/Empfänger 38 weiterleitet. Der implantatseitige HF-Sender/Empfänger 26 wird von der gleichen Energiequelle versorgt wie die anderen Implantatkomponenten, d.h. von einer Primärbatterie oder einem nachladbaren Akkumulator. Bei der voll implantierbaren Lösung gemäß Fig. 1 wird kein am Körper extern zu tragendes und damit sichtbares System benötigt.

Das Einwählen in das Telekommunikationsnetz sowie die Steuerung weiterer Telekommunikationsfunktionen kann beispielsweise mittels Spracheingabe erfolgen. In diesem Fall muß das Hörsystem 10 mit einem implantatseitigen Spracherkennungsmodul ausgestattet sein (in Fig. 1 nicht dargestellt), welches dann vorzugsweise in das Elektronikmodul 16 integriert ist.

Ferner kann das Hörsystem einen Speicher zu elektronischen Zwischenspeicherung von Telekommunikationsinhalten aufweisen (in Fig. 1 nicht dargestellt).

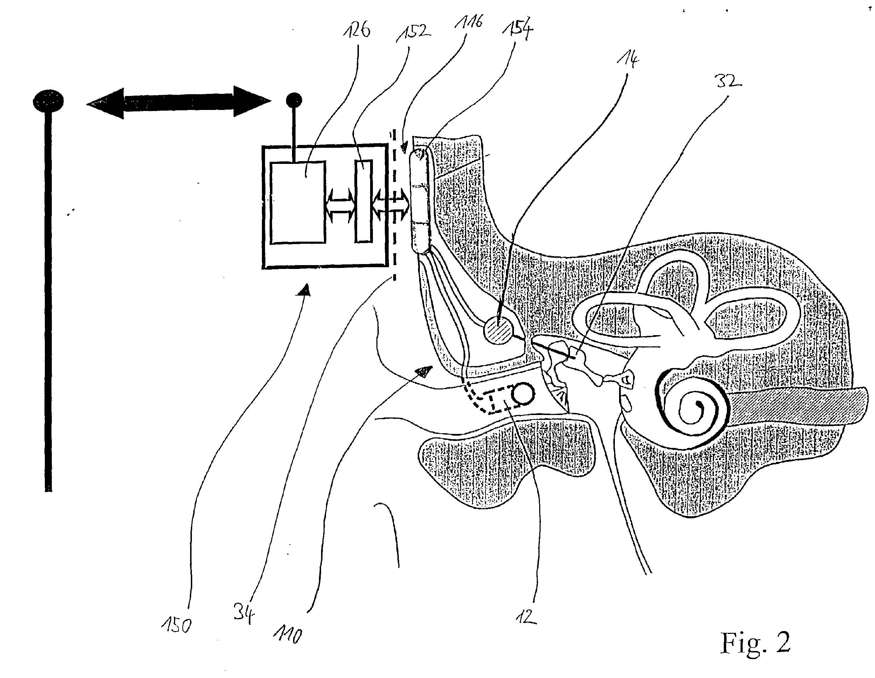

Fig. 2 zeigt eine alternative Ausgestaltung der Anbindung des Hörsystems von Fig. 1 an ein Telekommunikationsnetz. Der wesentliche Unterschied besteht darin, daß die Ankopplung an die HF-Strecke nicht über einen implantierten HF-Sender/Empfänger 26, sondern über eine am Kopf getragene Übertragungseinheit 150 erfolgt, welche einerseits einen HF-Sender/Empfänger 126 und andererseits eine bidirektionale Telemetrieschnittstelle 152 aufweist. Bei dieser Ausführungsform ist der implantatseitige HF-Sender/Empfänger 26 durch eine bidirektionale drahtlose Telemetriedatenschnittstelle 154 ersetzt, welche für den bidirektionalen Datenaustausch mit der Telemetrieschnittstelle 152 der am Kopf getragenen Übertragungseinheit 150 ausgebildet ist. Die drahtlose Telemetrie zwischen dem Implantat und der Übertragungseinheit 150 kann prinzipiell auch auf einer HF-Strecke basieren, vorteilhafterweise wird jedoch ein induktives Übertragungsverfahren gewählt, wobei die implantatseitige Telemetrieschnittstelle 154 beispielsweise von der Ladespule für den implantatseitigen Energiespeicher 24 und/oder von einer Fernbedienungs-Datenempfangsspule, die ohnehin im Implantat vorhanden sein können, gebildet wird. Weitere Möglichkeiten sind transkutane Infrarot- oder Ultraschallstrecken.

Die Übertragungseinheit 150 kann beispielsweise wie ein drahtloser Kopfhörer ausgestaltet sein, wie dies beispielsweise zur Hörunterstützung beim Fernsehen bekannt ist, und batterie- oder akkubetrieben sein. Der HF-Sender/Empfänger 126 steht in gleicher Weise mit einem HF-Datennetzknotenpunkt, d.h. mit einem stationären Großdistanz-HF-Sender/Empfänger 38, in bidirektionaler Kommunikation wie der Kleindistanz-Repeater 36 bei dem Ausführungsbeispiel gemäß Fig. 1.

Der Vorteil der Ausführungsform gemäß Fig. 2 besteht in dem geringeren Energiebedarf des Hörsystems aufgrund der Tatsache, daß kein implantatseitiger HF-Sender/Empfänger erforderlich ist. Die Übertragungseinheit 150 wirkt aufgrund der direkten Kommunikation mit dem Datennetzknotenpunkt 38 als mobiler Repeater und übernimmt insofern die Funktion des stationären Repeaters 36 bei der Ausführungsform von Fig. 1.

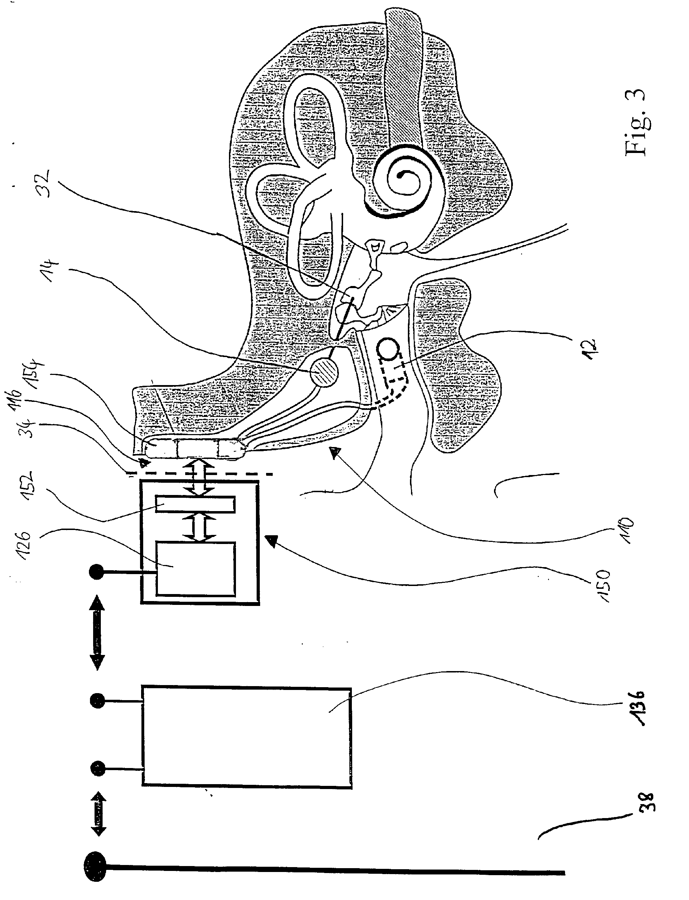

In Fig. 3 ist eine Abwandlung der Ausführungsform von Fig. 2 mit am Kopf getragener Übertragungseinheit 150 dargestellt. Dabei kommuniziert die Übertragungseinheit 150 nicht direkt mit dem Telekommunikations-Datennetzknotenpunkt 38, sondern über einen stationären Repeater 136, der die eigentliche HF-Ankopplung an das Telekommunikationsnetz vornimmt. In diesem Fall wirkt die am Kopf getragene Übertragungseinheit 150 nicht als Repeater, sondern als drahtlose Telemetrieschnittstelle zwischen dem Implantat und dem stationären Repeater 136. In diesem Fall kann die drahtlose Datenverbindung zwischen dem Kleindistanz-Repeater 136 und dem Sender/Empfänger 126 der Übertragungseinheit 150 über eine HF- oder eine Infrarotstrecke erfolgen, d.h. bei der Sender/Empfänger-Einheit 126 kann es sich um ein HF-System oder ein Infrarotsystem handeln. Die übrigen Systemkomponenten, insbesondere die bidirektionale Telemetrieverbindung zwischen der Übertragungseinheit 150 und dem Implantat, können analog zu Fig. 2 ausgebildet sein.

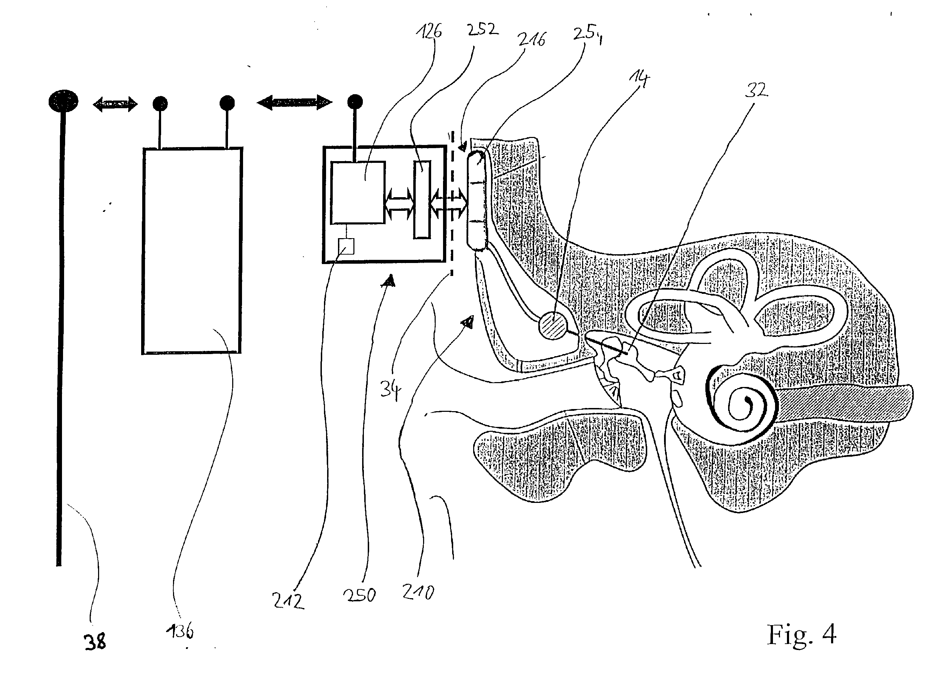

Bei der Ausführungsform gemäß Fig. 4 ist die Anbindung des Hörsystems an das Telekommunikationsnetz in der gleichen Weise wie in Fig. 3 verwirklicht, d.h. eine am Kopf getragene Übertragungseinheit 250 enthält einen Sender/Empfänger 126, der in bidirektionaler Kommunikation mit einem stationären Kleindistanz-Repeater 136 steht, welcher wiederum in bidirektionaler Kommunikation über eine HF-Strecke mit dem Telekommunikationsnetzknotenpunkt 38 steht. Im Gegensatz zu den Ausführungsformen gemäß Fig. 1 bis 3 ist jedoch das Hörsystem 210 nicht als vollimplantierbares System, sondern als teilimplantierbares System ausgebildet, bei welchem das Schallaufnehmermikrofon 212 sowie zweckmäßigerweise der größte Teil der Signalverarbeitungs/Steuerelektronik in der externen Übertragungseinheit 250 untergebracht sind. Das implantatseitige Elektronikmodul 216 kann dabei energetisch passiv ausgebildet sein und wie bei üblichen Teilimplantaten die Betriebsenergie und die Signaldaten transkutan empfangen und demodulieren, wobei dann kein implantatseitiger Energiespeicher 24 vorgesehen ist. Alternativ kann jedoch das Implantat wie bei den vorhergehenden Ausführungsformen eine aktive Energieversorgung in Form einer Primärbatterie oder eines Akkumulators enthalten. Als weiterer Unterschied zur Ausführungsform gemäß Fig. 3 ergibt sich, daß die transkutane Telemetrieverbindung zwischen der externen Telemetrieschnittstelle 252 und der implantatseitigen Telemetrieschnittstelle 254 lediglich unidirektional von der Übertragungseinheit 250 zu dem implantatseitigen Elektronikmodul 216 sein muß, da letzteres kein aufgenommenes Schallsignal erzeugt.

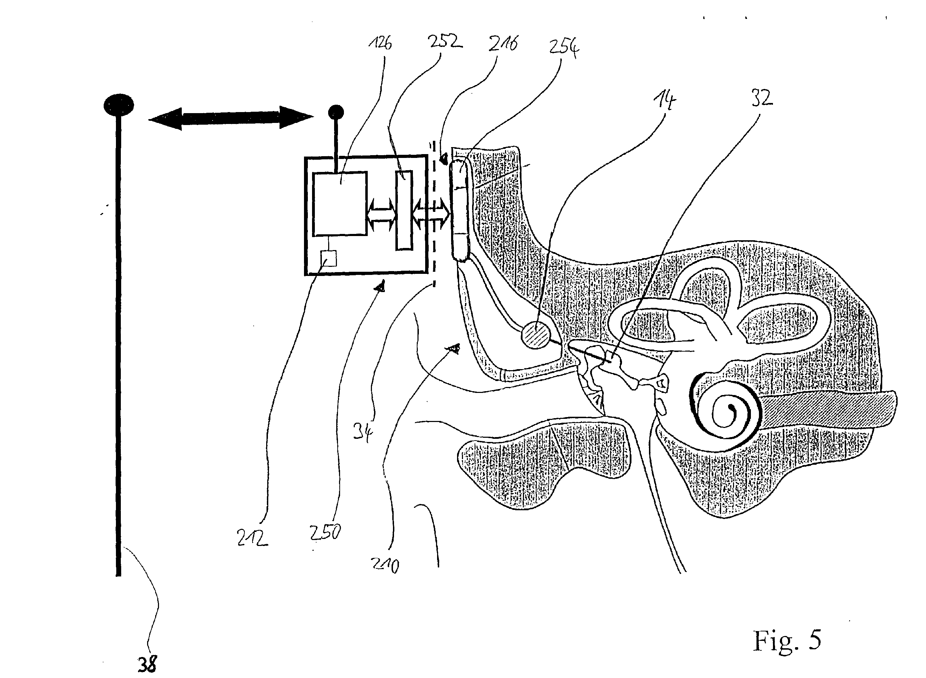

Fig. 5 zeigt eine Ausführungsform, die sich von der Ausführungsform gemäß Fig. 4 im wesentlichen dadurch unterscheidet, daß wie bei der Ausführungsform gemäß Fig. 2 auf einen stationären Kleindistanz-Repeater verzichtet wird und die Sende/Empfangs-Einheit 126 der am Kopf getragenen Übertragungseinheit 250 als HF-Sender/Empfänger zur direkten bidirektionalen Kommunikation mit dem Großdistanz-Telekommunikationsnetzknotenpunkt 38 ausgebildet ist und insofern als HF-Repeater wirkt.

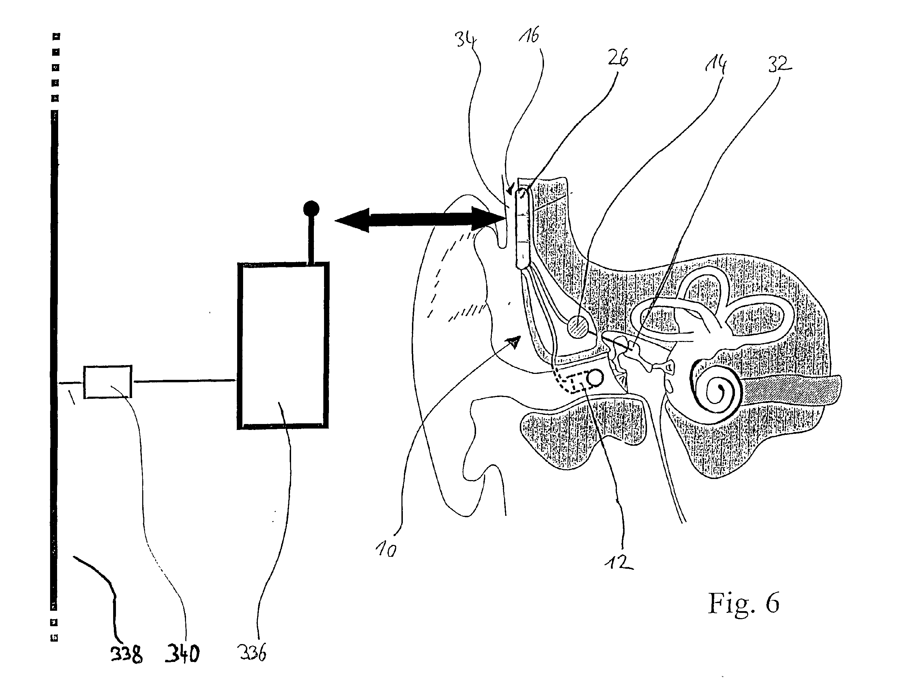

Fig. 6 zeigt eine Abwandlung der Ausführungsform gemäß Fig. 1, wobei der stationäre Kleindistanz-HF-Sender/Empfänger bzw. Repeater nicht für die Ankopplung an ein Telekommunikationsnetz über eine drahtlose HF-Strecke ausgebildet ist, sondern eine leitungsgebundene Ankopplung an ein leitungsgebundenes Telekommunikationsnetz 338 realisiert. Der Repeater 336 steht dabei wie in Fig. 1 in bidirektionaler drahtloser HF-Kommunikation mit dem Implantat 10. Die leitungsgebundene Ankopplung an das Telekommunikationsnetz 338 erfolgt eventuell über einen Adapter 340, der die jeweilige Umsetzung an die Hardwareauslegung des Telekommunikationsnetzes (Kabel, Lichtleitfaser, Stromversorgungsnetz etc.) vornimmt.

Das Konzept gemäß Fig. 6 kann natürlich auch für alle anderen Ausführungsformen verwendet werden, bei welchen ein stationärer Kleindistanz-Repeater vorgesehen ist.

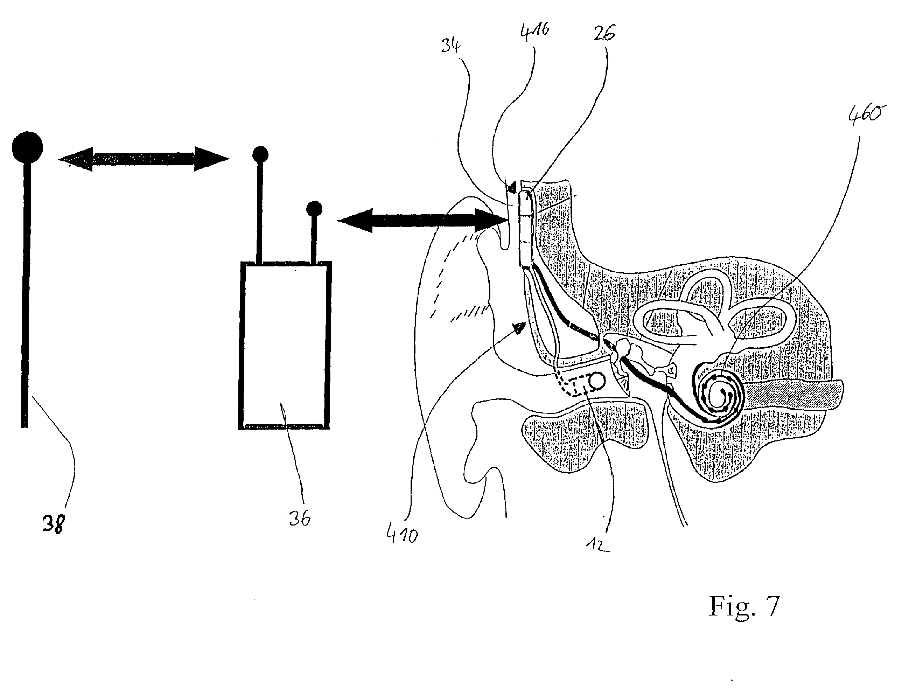

Fig. 7 zeigt ein voll implantierbares Hörsystem 410, welches jedoch statt eines elektromechanischen Wandlers zur mechanischen Stimulation der Ossikelkette oder zur direkten mechanischen Stimulation des Innenohrs eine Einheit zur elektrischen Stimulation des Innenohrs aufweist, bei welcher es sich um ein intercochleäres Multielektroden-Array 460 handelt, welches an sich bekannt ist und in üblicher Weise ausgeführt sein kann. Hinsichtlich der Anbindung an das Telekommunikationsnetz ist in Fig. 7 das Anbindungskonzept gemäß Fig. 1 dargestellt, bei welchem das Implantatelektronikmodul 416 einen HF-Sender/Empfänger 26 zur bidirektionalen Kommunikation mit einem stationären Kleindistanz-HF-Repeater 36 umfaßt, welcher wiederum in bidirektionaler Kommunikation mit einem Großdistanz-Telekommunikationsknotenpunkt 38 steht.

Die bisher geschilderten Ausführungsformen betrafen Hörsysteme zur bidirektionalen Anbindung an ein Telekommunikationsnetz, wobei das Hörsystem grundsätzlich sämtliche Funktionen eines Telekommunikationsendgerätes, wie z.B. eines Mobiltelefons, übernimmt.

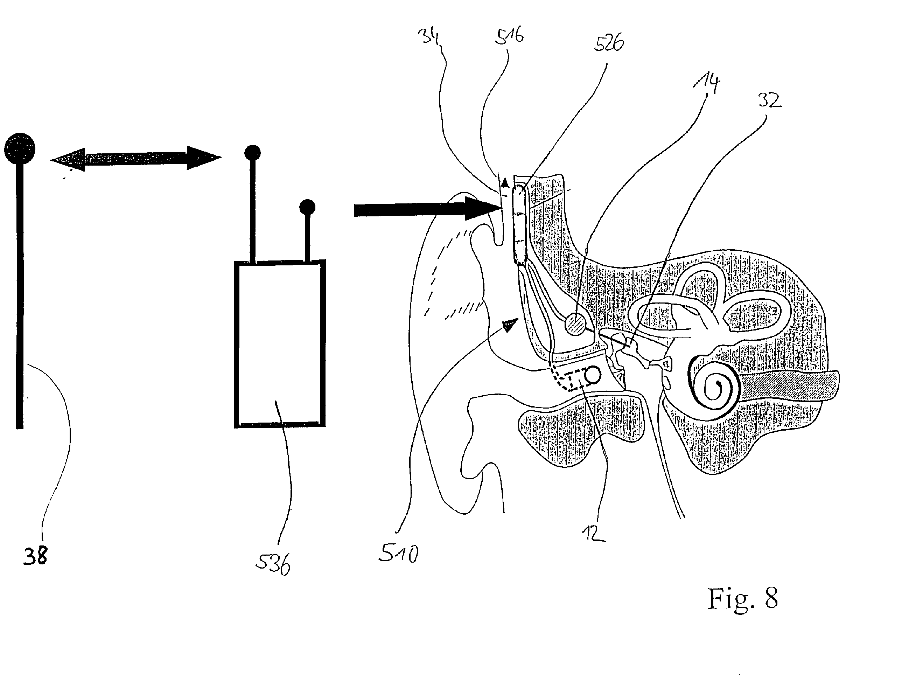

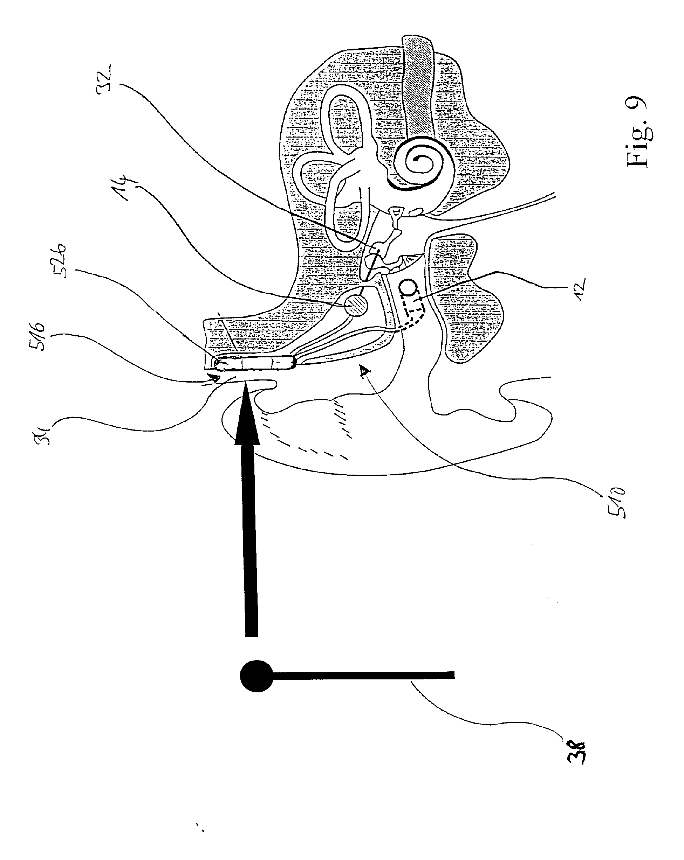

Die Figuren 8 und 9 zeigen Ausführungsformen für Hörsysteme, welche nur den implantatseitigen Empfang von sprachbasierten Nachrichten aus dem Telekommunikationsnetz erlauben. Die in Fig. 8 gezeigte Ausführungsform unterscheidet sich dabei von der Ausführungsform gemäß Fig. 1 im wesentlichen nur dadurch, daß in dem Elektronikmodul 516 statt eines HF-Sender/Empfängers nur ein HF-Empfänger 526 vorgesehen ist, welcher Signale von einem stationären HF-Kleindistanz-Repeater 536 empfängt, der in bidirektionaler Kommunikation mit einem stationären Großdistanz-HF-Sender/Empfänger 38 steht, der einen Telekommunikationsnetzknotenpunkt bildet. Das von dem HF-Empfänger 526 empfangene Telekommunikationssignal wird von diesem demoduliert und wie bei den vorhergehenden Ausführungsformen wird die so erhaltene Sprachinformation in den Audiosignalpfad des implantierten Hörsystems 510 eingespeist. Der stationäre Kleindistanz-HF-Sender/Empfänger 536 steht deshalb in bidirektionaler Kommunikation mit dem Knotenpunkt 38, um Empfangsbereitschaft und beispielsweise Empfangsquittierungen an das Telekommunikationsnetz zu übermitteln.

Die Ausführungsform gemäß Fig. 9 unterscheidet sich von der Ausführungsform gemäß Fig. 8 im wesentlichen dadurch, daß auf einen stationären HF-Sender/Empfänger als externe Zwischenstation verzichtet wird und die Datenübertragung rein unidirektional direkt von einem stationären Großdistanz-HF-Sender/Empfänger 38 (Telekommunikationsnetz-Knotenpunkt) direkt auf den implantatseitigen HF-Empfänger 526 erfolgt. Da hier kein Datenrückwärtspfad vorgesehen ist, kann in diesem Fall keine Empfangsbereitschaft bzw. Empfangsquittierung signalisiert werden.

Es versteht sich, daß die vorstehend im Zusammenhang mit bidirektionaler Anbindung an das Telekommunikationsnetz beschriebenen Hörsysteme grundsätzlich auch für eine unidirektionale, d.h. nur einen Empfang erlaubende Anbindung an das Telekommunikationsnetz ausgebildet sein können.

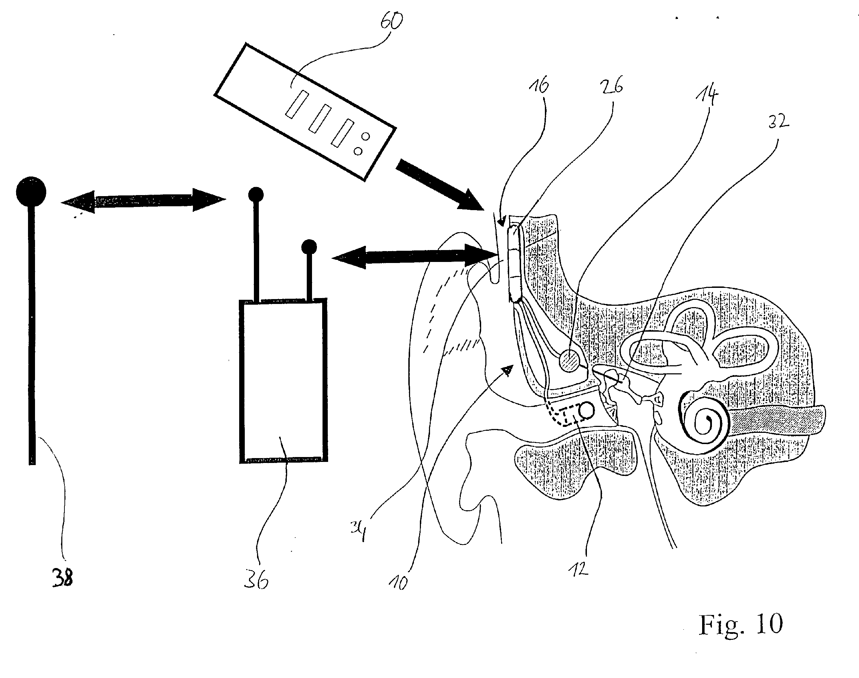

Fig. 10 zeigt eine Abwandlung der Ausführungsform gemäß Fig. 1, bei welcher zusätzlich eine drahtlose Fernbedienung 60 zur Steuerung der Telekommunikationsfunktionen, wie beispielsweise Einwählen in das Netz, Einstellung der Empfangslautstärke etc., des Implantats 10 vorgesehen ist. Die Kommunikation erfolgt dabei unidirektional von der Fernbedienung 60 auf das Elektronikmodul 16. Dabei kann beispielsweise die Kommunikation mittels einer HF-Strecke über den ohnehin vorhandenen HF-Sender/Empfänger 26 des Implantats 10 erfolgen. Alternativ kann die Kommunikation jedoch auch über eine induktive Strecke oder eine Infrarot- oder Ultraschallstrecke erfolgen. Wenn eine induktive Übertragung gewählt wird, kann beispielsweise die Ladespule und/oder eine Empfangsspule verwendet werden, die ohnehin im Implantat bereits vorhanden ist, um einen Datenaustausch bezüglich der eigentlichen Hörsystemfunktionen zu ermöglichen, z.B. Parameter-Einstellung oder Software-Aktualisierung, wie dies beispielsweise in der älteren EP-Patentanmeldung 99 115 256.2 beschrieben ist.

Falls das Implantat einen Speicher zur elektronischen Zwischenspeicherung von Telekommunikationsnachrichten enthält, kann die Fernbedienung 60 auch dazu dienen, solche zwischengespeicherten Nachrichten abzurufen.

Es versteht sich, daß eine solche Fernbedienung zur Steuerung der implantatseitigen Telekommunikationsfunktionen grundsätzlich auch bei allen anderen beschriebenen Ausführungsformen der Erfindung verwendet werden kann. Es versteht sich ferner, daß es sich bei dem teil- oder vollimplantierbaren Hörsystem auch um ein binaurales System handeln kann, wie es beispielsweise anhand der Fig. 5 der älteren EP-Patentanmeldung 99 115 256.2 erläutert ist.

## Patentansprüche

1. Teil- oder vollimplantierbares Hörsystem, mit einer Einheit (14, 30; 460) für die elektromechanische Stimulation des Mittel- oder Innenohres oder die direkte elektrische Stimulation des Innenohres, **dadurch gekennzeichnet, dass** das Hörsystem eine Einrichtung (26, 126, 154, 526) zur direkten Anbindung des Hörsystems an Telekommunikationsnetze ohne die Verwendung von Telekommunikationsendgeräten umfasst.

2. Hörsystem nach Anspruch 1, **dadurch gekennzeichnet, dass** das Hörsystem mit einem Schallaufnehmer (12, 212) versehen ist.

3. Hörsystem nach Anspruch 2, **dadurch gekennzeichnet, dass** die Einrichtung (26, 126, 154) zur bidirektionalen Anbindung ausgebildet ist.

4. Hörsystem nach Anspruch 3, **dadurch gekennzeichnet, dass** die Anbindungseinrichtung eine Einrichtung (26, 126, 154) zum drahtlosen Senden und Empfangen von Signalen aufweist.

5. Hörsystem nach Anspruch 4, **dadurch gekennzeichnet, dass** das Hörsystem eine am Kopf zu tragende Übertragungseinheit (250) aufweist, die den Schallaufnehmer (212), die Sende-Empfangs-Einrichtung (126) sowie eine Sendeeinrichtung (252) zur unidirektionalen Kommunikation über einen implantierten Empfänger (254) mit implantierten Komponenten (216) des Hörsystems aufweist.

6. Hörsystem nach Anspruch 5, **dadurch gekennzeichnet, dass** die Kommunikation zwischen der Sendeeinrichtung (252) und dem implantierten Empfänger (254) induktiv oder mittels einer transkutanen Infrarot- oder Ultraschallstrecke erfolgt.

7. Hörsystem nach einem der Ansprüche 4 bis 6, **dadurch gekennzeichnet, dass** die Sende-Empfangs-Einrichtung als HF-Sender-Empfänger-Einrichtung (26, 126) ausgebildet ist.

8. Hörsystem nach Anspruch 7, **dadurch gekennzeichnet, dass** die HF-Sender-Empfänger-Einrichtung (26, 126) zur Kleindistanz-Kommunikation mit einer stationären Repeater-Einheit (36, 336) ausgebildet ist.

9. Hörsystem nach Anspruch 7, sofern auf Anspruch 4 oder 5 rückbezogen, **dadurch gekennzeichnet, dass** die HF-Sender-Empfänger-Einrichtung (126) zur Großdistanz-Kommunikation mit einem HF-Telekommunikationsnetz-Knotenpunkt (38) ausgebildet ist.

10. Hörsystem nach Anspruch 4, **dadurch gekennzeichnet, dass** die Sende-Empfangs-Einrichtung (154) zur Kommunikation mit einer am Kopf zu tragenden Übertragungs-Einheit (150) ausgebildet ist.

11. Hörsystem nach Anspruch 10, **dadurch gekennzeichnet, dass** die Kommunikation induktiv (vorzugsweise über Empfangsspule für Energiespeicher oder Ferndatenaustauschempfangsspule) oder mittels einer transkutanen Infrarot- oder Ultraschallstrecke erfolgt.

12. Hörsystem nach Anspruch 10 oder 11, **dadurch gekennzeichnet, dass** die am Kopf zu tragende Übertragungs-Einheit (150) zur Großdistanz-Kommunikation mit einem HF-Telekommunikationsnetz-Knotenpunkt (38) ausgebildet ist.

13. Hörsystem nach Anspruch 10 oder 11, **dadurch gekennzeichnet, dass** die am Kopf zu tragende Übertragungs-Einheit (150) zur Kleindistanz-Kommunikation mit einer stationären Repeater-Einheit (136) ausgebildet ist.

14. Hörsystem nach Anspruch 8 oder 13, **dadurch gekennzeichnet, dass** die stationäre Repeater-Einheit (36, 136) zur Großdistanz-Kommunikation mit einem HF-Telekommunikationsnetz-Knotenpunkt (38) ausgebildet ist.

15. Hörsystem nach Anspruch 8 oder 13, **dadurch gekennzeichnet, dass** die stationäre Repeater-Einheit (336) zur leitungsgebundenen Ankopplung an ein leitungsgebundenes Telekommunikationsnetz (338) ausgebildet ist.

16. Hörsystem nach Anspruch 4 oder 10 bis 13, **dadurch gekennzeichnet, dass** die Sende-Empfangs-Einrichtung (26, 154) implantierbar ist.

17. Hörsystem nach Anspruch 16, **dadurch gekennzeichnet, dass** das Hörsystem ein Elektronikmodul (16, 116) aufweist, um aus dem von dem Schallaufnehmer (12) erfassten Signal das Eingangssignal für die Einheit (14, 30; 460) für die elektromechanische Stimulation des Mittel- oder Innenohres oder die direkte elektrische Stimulation des Innenohres zu erzeugen.

18. Hörsystem nach Anspruch 17, **dadurch gekennzeichnet, dass** das Elektronikmodul (16, 116) einen drahtlos aufladbaren Speicher (24) für elektrische Energie umfasst.

19. Hörsystem nach Anspruch 17 oder 18, **dadurch gekennzeichnet, dass** die Sende-Empfangs-Einrichtung (26, 154) in das Elektronikmodul (16, 116) integriert ist.

20. Hörsystem nach einem der Ansprüche 16 bis 19, **dadurch gekennzeichnet, dass** alle Komponenten des Hörsystems implantierbar sind.

21. Hörsystem nach Anspruch 20, **dadurch gekennzeichnet, dass** der Schallaufnehmer ein implantiertes Mikrofon (12) ist, das den Schall im Gehörgang abgreift.

22. Hörsystem nach Anspruch 1 oder 2, **dadurch gekennzeichnet, dass** die Einrichtung (526) zur unidirektionalen Anbindung an das Telekommunikationsnetz ausgebildet ist, wobei die Kommunikation vom Telekommunikationsnetz zum Hörsystem erfolgt.

23. Hörsystem nach Anspruch 22, **dadurch gekennzeichnet, dass** die Anbindungseinrichtung eine Einrichtung (526) zum drahtlosen Empfangen von Signalen aufweist.

24. Hörsystem nach Anspruch 23, **dadurch gekennzeichnet, dass** die Empfangs-Einrichtung als HF-Empfänger-Einrichtung (526) ausgebildet ist.

25. Hörsystem nach Anspruch 24, **dadurch gekennzeichnet, dass** die HF-Empfänger-Einrichtung (526) zum Kleindistanz-Empfang von HF-Signalen von einer stationären Repeater-Einheit (536) ausgebildet ist.

26. Hörsystem nach Anspruch 25, **dadurch gekennzeichnet, dass** die stationäre Repeater-Einheit (526) zur bidirektionalen Großdistanz-Kommunikation mit einem HF-Telekommunikationsnetz-Knotenpunkt (38) ausgebildet ist.

27. Hörsystem nach Anspruch 24, **dadurch gekennzeichnet, dass** die HF-Empfänger-Einrichtung (526) zum Großdistanz-Empfang von HF-Signalen von einem HF-Telekommunikationsnetz-Knotenpunkt (38) ausgebildet ist.

28. Hörsystem nach einem der vorhergehenden Ansprüche, **dadurch gekennzeichnet, dass** die Stimulationseinheit einen elektromechanischen Wandler (14) und ein von diesem angetriebenes starres Koppelelement (30) aufweist, das an ein Mittelohrossikel (32) oder an das ovale Fenster angekoppelt ist.

29. Hörsystem nach einem der Ansprüche 1 bis 27, **dadurch gekennzeichnet, dass** die Stimulationseinheit (460) mehrere in die Cochlea implantiertbare Elektroden aufweist.

30. Hörsystem nach einem der vorhergehenden Ansprüche, **dadurch gekennzeichnet, dass** das Hörsystem einen Speicher zur elektronischen Zwischenspeicherung von Telekommunikationsinhalten aufweist.

31. Hörsystem nach einem der vorhergehenden Ansprüche, **dadurch gekennzeichnet, dass** das Hörsystem ein Spracherkennungsmodul zum sprachgesteuerten Einwählen in das Telekommunikationsnetz umfasst.

32. Hörsystem nach einem der vorhergehenden Ansprüche, **dadurch gekennzeichnet, dass** das Hörssystem zur Kommunikation mit einer drahtlosen Fernbedienung (60) zur Steuerung von Telekommunikationsfunktionen, beispielsweise Einwahl in das Telekommunikationsnetz, Abrufen von zwischengespeicherten Nachrichten, oder Lautstärkeeinstellung von Sprachnachrichten, des Hörsystems ausgebildet ist.

33. Hörsystem nach einem der vorhergehenden Ansprüche, **dadurch gekennzeichnet, dass** das Hörsystem für die Rehabilitation einer Hörstörung, insbesondere des Mittelohrs und/oder Innenohrs, ausgebildet ist.

34. Hörsystem nach einem der Ansprüche 1 bis 32, **dadurch gekennzeichnet, dass** das Hörsystem für einen Benutzer ohne Hörstörung ausgelegt ist.

35. Hörsystem nach einem der vorhergehenden Ansprüche, **dadurch gekennzeichnet, dass** das Hörsystem als binaurales System ausgebildet ist.
